# EUROPEAN PATENT APPLICATION

(11) **EP 4 015 006 A1**
(43) Date of publication of application: **22.06.2022**
(21) Application number: 21213704.6
(22) Date of filing: 10.12.2021
(51) Int. Cl.: A61L 2/14, B65G 17/06, B65G 41/00

(54) **CONVEYER BELT TYPE PLASMA STERILIZER**

(30) Priority: 18.12.2020 KR 20200178682
(71) Applicant: Lee, Chang Hoon, Seoul 03477 (KR)
(72) Inventor: Lee, Chang Hoon, Seoul 03477 (KR)
(74) Representative: Faraldi, Marco

(57) **Abstract**

A conveyer belt type plasma sterilizer is provided, including a first chamber having a shape of a square cylinder with a closed ceiling and an open bottom, the first chamber including a plurality of first plasma ejection tools on one sidewall, a second chamber having a shape of a square cylinder, in which the second chamber is formed to be longer than the first chamber to surround the first chamber when viewed along a longitudinal direction of the first chamber, and includes a plurality of gas inlet holes below the first chamber and a plurality of gas outlet holes on the ceiling of the first chamber, a first conveyer belt at one side of the second chamber, extending from an outside of the second chamber to a vicinity of the second chamber, a second conveyer belt starting near the first conveyer belt, extending through the second chamber and the first chamber, and toward the other side of the second chamber, and a plurality of second plasma ejection tools below the second chamber, facing each of the gas inlet holes of the second chamber.

## Description

### BACKGROUND

### Technical field

The present disclosure relates to a conveyer belt type plasma sterilizer which exposes an object, which may be at least one of industrial and agricultural products, pharmaceuticals, and toys for children, to plasma to environmentally and simply clean the surface of the object regardless of the shape of the object and increase the shelf life of the object.

### Background Art

In general, plasma refers to a fourth state of matter which consists of ions, electrons, radicals, and the like generated by an externally applied electric field, and neutral particles, and which is macroscopically electrically neutral. By using ions, electrons, radicals, and the like, the plasma is widely used in the fields of surface modification, etching, coating or sterilization, disinfection, ozone generation, dyeing, wastewater and tap water purification, air purification, high-brightness lamps, and the like.

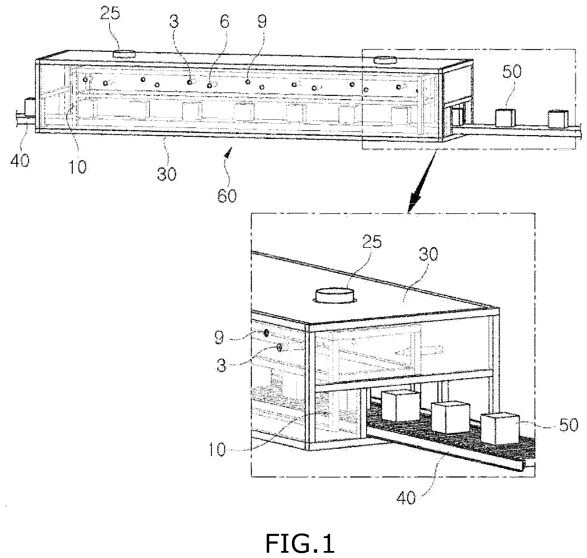

For the simplicity of the description, the plasma is described herein by focusing on use for sterilization or disinfection purposes. FIG. 1 shows related conveyer belt type plasma sterilization equipment 60. The plasma sterilization equipment 60 includes a first chamber 10, a second chamber 30 surrounding the first chamber 10, and a conveyer belt 40 passing through the first and second chambers 10 and 30.

The first chamber 10 includes plasma irradiation guns 3 and 6 on one and the other sidewalls, and first outlet holes 9 between the plasma irradiation guns 3 and 6 on one and the other sidewalls. The second chamber 30 includes second outlet holes 25 in the ceiling on the first chamber 10.

After agricultural products 50 are placed on the conveyer belt 40 and while the conveyer belt 40 is driven, the plasma sterilization equipment 60 irradiates the inside of the first chamber 10 with a plasma jet (not illustrated) by using the plasma irradiation guns 3 and 6 to supply a sterilizing gas of the plasma jet to the inside of the first chamber 10.

The first and second chambers 10 and 30 include first and second entrances at places where the agricultural products 50 enter, and first and second exits at places where the agricultural products 50 are discharged. The sterilizing gas has a first gas path through the first outlet holes 9 of the first chamber 10 and the second outlet holes 25 of the second chamber 30, a second gas path through the first entrance of the first chamber 10 and the second entrance of the second chamber 30, and a third gas path through the first exit of the first chamber 10 and the second exit of the second chamber 30.

Meanwhile, the first gas path is shorter than the second and third gas paths and is formed before the second and third gas paths in the first and second chambers 10 and 30. Accordingly, the presence of the first path results in a lowered internal pressure of the first and second chambers 10 and 30. In addition, while the conveyer belt 40 moves the agricultural products 50, the agricultural products 50 cause external air to be introduced into the first and second entrances of the first and second chambers 10 and 30.

This is because the presence of the first path results in a reduced difference between the internal pressure of the second chamber 30 and the external pressure of the second chamber 30. Accordingly, the agricultural products 50 are not exposed to a constant concentration of sterilizing gas along the first and second chambers 10 and 30 and as a result, the surface of the agricultural product 50 is not modified, and the agricultural products 50 are severely affected by the external environment over time and presented to the buyer with reduced quality.

Meanwhile, the conveyer belt type plasma sterilization equipment is also similarly disclosed as a related art in Korean Patent Publication No. 10-1049971.

### CITATION LIST

### PATENT LITERATURE

(PTL 1) Korean Patent Publication No. 10-1049971

### SUMMARY

### Technical Problem

The present disclosure has been made in order to solve the problems of the related art, and an object of the present disclosure is to provide a conveyer belt type plasma sterilizer suitable for blocking the inflow of external air from the outside toward the inside of the second chamber by increasing the difference between the internal pressure of the second chamber and the external pressure of the second chamber during driving of the conveyer belt and the plasma guns, thus exposing an object, which may be at least one of industrial and agricultural products, pharmaceuticals, and toys for children, to the plasma to environmentally and simply clean the surface of the object regardless of the shape of the object and increase the shelf life of the object.

### Technical Solution

A conveyer belt type plasma sterilizer is provided, which may include a first chamber having a shape of a square cylinder with a closed ceiling and an open bottom, the first chamber including a plurality of first plasma ejection tools on one sidewall, a second chamber having a shape of a square cylinder, in which the second chamber may be formed to be longer than the first chamber to surround the first chamber when viewed along a longitudinal direction of the first chamber, and include a plurality of gas inlet holes below the first chamber and a plurality of gas outlet holes on the ceiling of the first chamber, a first conveyer belt at one side of the second chamber, extending from an outside of the second chamber to a vicinity of the second chamber, a second conveyer belt starting near the first conveyer belt, extending through the second chamber and the first chamber, and toward the other side of the second chamber, and a plurality of second plasma ejection tools below the second chamber, facing each of the gas inlet holes of the second chamber, in which the first conveyer belt may include a mesh type or non-mesh type belt, and the second conveyer belt may include a mesh type belt.

Each of the first plasma ejection tools may either include a plasma nozzle and a nozzle head fixedly coupled in sequence to a plasma generator which penetrates the one sidewall of the first chamber and is exposed to an inside of the first chamber. or include a plasma nozzle and a nozzle head rotationally coupled in sequence to the plasma generator.

Each of the first plasma ejection tools may include a plasma nozzle and a nozzle head coupled in sequence to a plasma generator which is positioned on the one sidewall of the first chamber so as to extend from the second chamber toward the first chamber, and may receive power from the outside of the second chamber through a cable to transform an air into a first plasma in the plasma generator, receive a compressed air from the outside of the second chamber to the plasma generator, and pass the first plasma and the compressed air through the plasma nozzle and the nozzle head to eject a first plasma jet from the nozzle head.

Each of the first plasma ejection tools may include a plasma nozzle and a nozzle head coupled in sequence to a plasma generator which is positioned on the one sidewall of the first chamber so as to extend from the second chamber toward the first chamber, and may irradiate an inside of the first chamber with a first plasma jet from the nozzle head to supply sterilizing gas of the first plasma jet to the first chamber to the inside of the first chamber during driving of the plurality of first plasma ejection tools, and the sterilizing gas may include ozone and active oxygen.

The first chamber may include a first entrance open toward the one side of the second chamber, and a first exit open toward the other side of the second chamber, in which the first chamber may be irradiated with a first plasma jet of each of the first plasma ejection tools through the first chamber and a second plasma jet of each of the second plasma ejection tools through the second chamber, and diffuse a first sterilizing gas of the first plasma jet and a second sterilizing gas of the second plasma jet toward the first entrance and the first exit of the first chamber, and the first sterilizing gas or the second sterilizing gas may include ozone and active oxygen.

The first chamber may include a first entrance open toward the one side of the second chamber, and a first exit open toward the other side of the second chamber, and further include at least one ozone concentration measuring sensor on the other sidewall of the first chamber between the first entrance and the first exit of the first chamber, and a control unit positioned on the outside of the second chamber and electrically connected to the at least one ozone concentration measuring sensor through the second chamber to display a measured value of the at least one ozone concentration measuring sensor on a monitor, in which the at least one ozone concentration measuring sensor may measure ozone concentrations from first and second sterilizing gases diffused into the first chamber through a first plasma jet of each of the first plasma ejection tools and a second plasma jet of each of the second plasma ejection tools.

When the first chamber is irradiated on the inside of the first chamber with a first plasma jet from each of the first plasma ejection tools and a first sterilizing gas of the first plasma jet fills the inside the first chamber, the second chamber may be irradiated at the gas inlet of the second chamber with a second plasma jet of each of the second plasma ejection tools and the second chamber may supply a second sterilizing gas of the second plasma jet to the inside of the first chamber through the gas inlet holes of the second chamber.

The first chamber or the second chamber may include a first entrance or a second entrance open toward the one side of the second chamber, and a first exit or a second exit open toward the other side of the second chamber, and when the first plasma ejection tools or the second plasma ejection tools supply a first sterilizing gas of a first plasma jet or a second sterilizing gas of a second plasma jet to an inside of the first chamber, the second chamber may be filled with the first sterilizing gas and the second sterilizing gas diffused from the first entrance of the first chamber towards the second entrance of the second chamber, and from the first exit of the first chamber toward the second exit of the second chamber, and may discharge the first sterilizing gas and the second sterilizing gas from an inside of the second chamber toward the first entrance of the second chamber, from the inside of the second chamber toward the second exit of the second chamber, and from the inside of the second chamber toward each of the gas outlet holes of the second chamber.

When each of the first plasma ejection tools or each of the second plasma ejection tools supplies a first sterilizing gas of a first plasma jet or a second sterilizing gas of a second plasma jet to an inside of the first chamber, the second chamber may include a filter in each gas outlet hole of the second chamber, and filter the first sterilizing gas and the second sterilizing gas through the filter, and discharge the filtered first sterilizing gas and the filtered second sterilizing gas toward the outside of the second chamber.

When viewed in a longitudinal direction of the second chamber, the first conveyer belt may be spaced apart from the second conveyer belt and positioned at a same horizontal level as the second conveyer belt or positioned at a higher vertical level than the second conveyer belt.

The first conveyer belt may include a plurality of first support structures below the first conveyer belt, and each of the first support structure may support both edges of the first conveyer belt in a width direction of the first conveyer belt.

The second conveyer belt may be rotated in a same rotational direction as the first conveyer belt, may be formed to be smaller than a width of the first chamber, and may cover each gas inlet hole of the second chamber while passing through the first chamber and the second chamber.

The second conveyer belt may be irradiated with a second plasma jet of each of the second plasma ejection tools through each gas inlet hole of the second chamber, and may diffuse a second sterilizing gas of the second plasma jet toward the first chamber through the mesh type belt.

The second chamber may include a second entrance open toward the one side of the second chamber, and a second exit open toward the other side of the second chamber, and the second conveyer belt may include an object detection sensor positioned near the second entrance or the second exit of the second chamber, and a control unit positioned on the outside of the second chamber and electrically connected to the object detection sensor to display a measured value of the object detection sensor on a monitor, in which the object detection sensor may detect an object positioned on the first conveyer belt or the second conveyer belt, and a rotation of the second conveyer belt may be performed when detecting the object or the rotation of the second conveyer belt may be stopped when not detecting the object.

Each of the second plasma ejection tools may include a plasma nozzle and a nozzle head rotationally coupled in sequence to a plasma generator positioned below the second chamber so as to extend from the outside of the second chamber toward the gas inlet holes of the second chamber, and may receive power from the outside of the second chamber through a cable to transform an air into a plasma in the plasma generator, receive a compressed air from the outside of the second chamber to the plasma generator, and pass the plasma and the compressed air through the plasma nozzle and the nozzle head to eject a plasma jet from the nozzle head.

The second chamber and the second conveyer belt may include a plurality of second support structures below the second chamber and the second conveyer belt when viewed in a longitudinal direction of the second chamber, and each of the second support structures may support both edges of the second chamber or the second conveyer belt in a width direction of the second chamber or the second conveyer belt.

When each of the first plasma ejection tools and each of the second plasma ejection tools supplies a first sterilizing gas of the first plasma jet or a second sterilizing gas of the second plasma jet to the inside of the first chamber, the first conveyer belt and the second conveyer belt may convey an object from the first conveyer belt toward the second conveyer belt during driving of the first conveyer belt and the second conveyer belt, and inside the first chamber, the object may be positioned below the plurality of first plasma ejection tools, and may be exposed to the first sterilizing gas and the second sterilizing gas on the second conveyer belt.

When each of the first plasma ejection tools and each of the second plasma ejection tools supplies a first sterilizing gas of the first plasma jet or a second sterilizing gas of the second plasma jet to the inside of the first chamber, the first sterilizing gas and the second sterilizing gas may increase the internal pressure of the first chamber and the second chamber compared to the atmospheric pressure on the outside of the second chamber to block the inflow of external air from the outside of the second chamber toward the inside of the second chamber.

The second chamber or the second conveyer belt may include a plurality of second support structures below the second chamber or the second conveyer belt when viewed along a longitudinal direction of the second chamber, and the plurality of second support structures may include a support plate positioned between the plurality of second support structures below the second chamber and fixed to the plurality of second support structures to collect foreign substances falling from the object through the gas inlet holes of the second chamber while the object is moved on the second conveyer belt.

### Advantageous Effects

The conveyer belt type plasma sterilizer according to the present disclosure includes a second chamber surrounding a first chamber, a first conveyer belt at one side of the second chamber, and a second conveyer belt passing through the first and second chambers, and removes the outlet hole of the related art from the sidewall of the first chamber, allows the first chamber and the second chamber to be in fluid communication through the second conveyer belt, and places a plurality of first plasma ejection tools inside the first chamber and a plurality of second plasma ejection tools below the second chamber, so that, in order to expose an object, which may be at least one of industrial and agricultural products, pharmaceuticals, and toys for children, to plasma to environmentally and simply clean the surface of the object regardless of the shape of the object and increase the shelf life of the object, the external pressure of the second chamber is made smaller than the internal pressure of the second chamber during driving of the conveyer belt and each of the first plasma ejection tools and each of the second plasma ejection tools, thereby blocking the inflow of external air from the outside toward the inside of the second chamber even when the object is transferred from the first conveyer belt toward the second conveyer belt.

### BRIEF DESCRIPTION OF THE DRAWINGS

FIG. 1 is a schematic diagram illustrating conveyer belt type plasma sterilization equipment according to the related art.
FIG. 2 is a schematic diagram illustrating a conveyer belt type plasma sterilizer according to the present disclosure.
FIG. 3 is a schematic diagram illustrating a positional relationship between a second chamber, a second conveyer belt, and a second plasma ejection tool in the conveyer belt type plasma sterilizer of FIG. 2.
FIG. 4 is a schematic diagram illustrating relative rotation of a plasma nozzle and a nozzle head with respect to a plasma generator in the second plasma ejection tool of FIG. 3.
FIG. 5 is a schematic diagram illustrating the plasma nozzle and nozzle head of FIG. 4, in which a plasma jet is ejected from the nozzle head during rotation of the plasma nozzle and the nozzle head.

### DETAILED DESCRIPTION OF THE INVENTION

The details of the present disclosure are described with reference to the accompanying drawings which illustrate specific embodiments in which the present disclosure may be practiced by way of illustration. These embodiments are described in sufficient detail to enable those skilled in the art to practice the present disclosure. It should be understood that various embodiments of the present disclosure are different from each other but need not be mutually exclusive. For example, certain shapes, structures, and characteristics described herein relate to one embodiment and may be implemented in other embodiments without departing from the spirit and scope of the present disclosure. In addition, it should be understood that the location or arrangement of each of the components within each disclosed embodiment may be changed without departing from the spirit and scope of the present disclosure. Accordingly, the detailed description provided below is not intended to be construed as limiting, and the scope of the present disclosure, if properly described, is limited only by the appended claims, along with all scope equivalents to those as claimed. In the drawings, like reference numerals refer to the same or similar functions in various aspects, and the length, area, thickness, and the like, and the shape thereof may be represented in an exaggerated manner.

Hereinafter, in order to enable those skilled in the art to easily practice the present disclosure, preferred embodiments of the present disclosure will be described in detail with reference to the accompanying drawings.

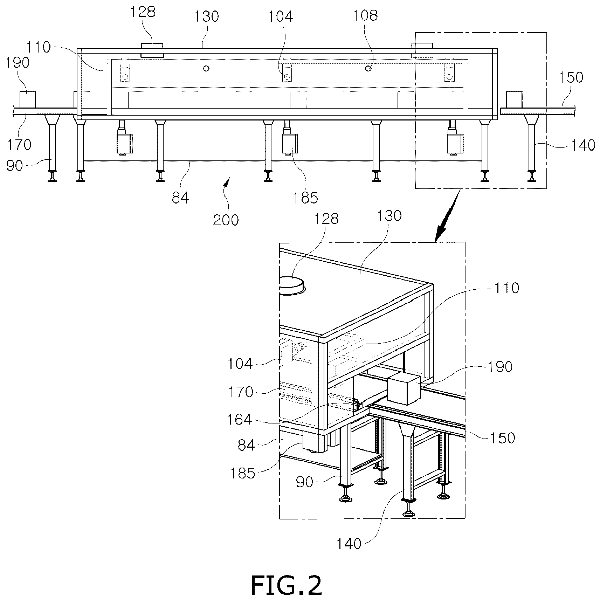

FIG. 1 is a schematic diagram illustrating conveyer belt type plasma sterilization equipment according to the related art, and FIG. 2 is a schematic diagram illustrating a conveyer belt type plasma sterilizer according to the present disclosure.

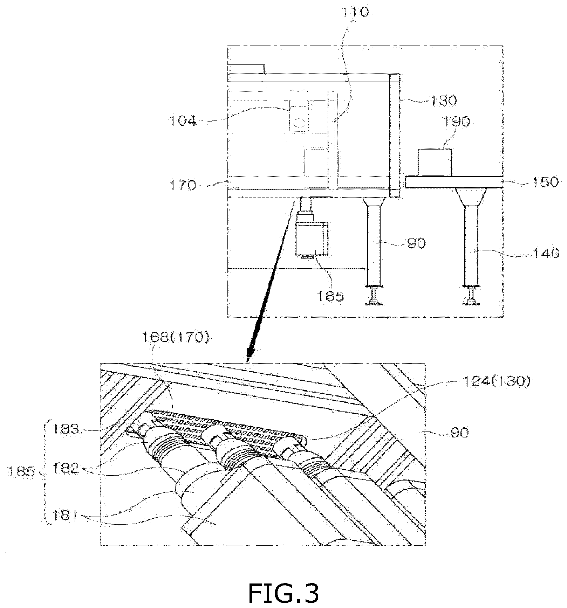

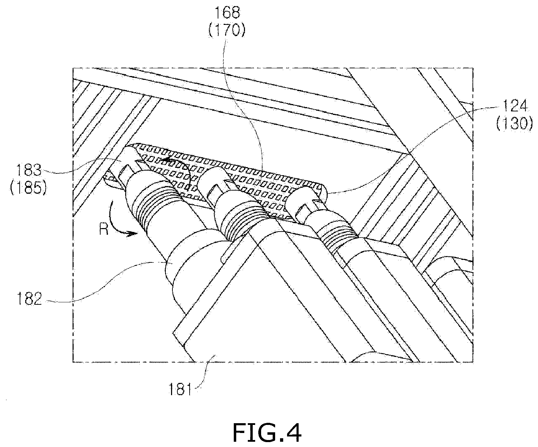

FIG. 3 is a schematic diagram illustrating a positional relationship between a second chamber, a second conveyer belt, and a second plasma ejection tool in the conveyer belt type plasma sterilizer of FIG. 2, and FIG. 4 is a schematic diagram illustrating relative rotation of a plasma nozzle and a nozzle head with respect to a plasma generator in the second plasma ejection tool of FIG. 3.

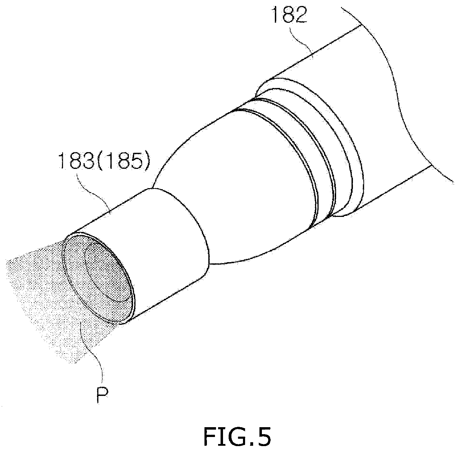

In addition, FIG. 5 is a schematic diagram illustrating the plasma nozzle and nozzle head of FIG. 4, in which a plasma jet is ejected from the nozzle head during rotation of the plasma nozzle and the nozzle head.

Referring to FIGS. 1 to 5, a conveyer belt type plasma sterilizer 200 according to the present disclosure includes a first chamber 110, a second chamber 130, a first conveyer belt 150 and a second conveyer belt 170, and a plurality of second plasma ejection tools 185, as illustrated in FIG. 1.

When viewed schematically, the first chamber 110 includes a plurality of first plasma ejection tools 104 on one sidewall of the first chamber 110, and have a shape of a square cylinder with a closed ceiling and an open bottom. As illustrated in FIGS. 2 to 4, the second chamber 130 is formed to be longer than the first chamber 110 and surrounds the first chamber 110 when viewed along the longitudinal direction of the first chamber 110, and includes a plurality of gas inlet holes 124 below the first chamber 110 and a plurality of gas outlet holes 128 on the ceiling of the first chamber.

As illustrated in FIG. 1, the first conveyer belt 150 at one side of the second chamber 130 extends from the outside of the second chamber 130 to the vicinity of the second chamber 130. As illustrated in FIG. 1, the second conveyer belt 170 starts near the first conveyer belt 150 and extends through the second chamber 130 and the first chamber 110, and towards the other side of the second chamber 130.

The plurality of second plasma ejection tools 185 are below the second chamber 130, facing each gas inlet hole 124 of the second chamber 130. The first conveyer belt 150 includes a mesh type or non-mesh type belt, and the second conveyer belt 170 includes a mesh type belt as illustrated in FIGS. 3 and 4.

In detail, referring to FIG. 3, each of the first plasma ejection tools 104 either includes a plasma nozzle and a nozzle head fixedly coupled in sequence to a plasma generator which penetrates the one sidewall of the first chamber 110 and is exposed to the inside of the first chamber 110, or includes a plasma nozzle and a nozzle head rotationally coupled in sequence to the plasma generator.

Each of the first plasma ejection tools 104 includes the plasma nozzle and the nozzle head coupled in sequence to the plasma generator positioned on one sidewall of the first chamber 110 so as to extend from the second chamber 130 toward the first chamber 110, and receives power from the outside of the second chamber 130 through a cable (not illustrated) to transform the air into the first plasma in the plasma generator, receives compressed air from the outside of the second chamber 130 to the plasma generator, and passes the first plasma and the compressed air through the plasma nozzle and the nozzle head to eject a first plasma jet from the nozzle head.

Each of the first plasma ejection tools 104 includes the plasma nozzle and the nozzle head coupled in sequence to the plasma generator positioned on one sidewall of the first chamber 110 so as to extend from the second chamber 130 toward the first chamber 110, and irradiates the inside of the first chamber 110 with a first plasma jet from the nozzle head to supply sterilizing gas of the first plasma jet to the first chamber 110 to the inside of the first chamber 110 during the driving of the plurality of first plasma ejection tools 104. In an example, the sterilizing gas includes ozone and active oxygen.

Referring to FIGS. 2 and 3, the first chamber 110 includes a first entrance open toward the one side of the second chamber 130, and a first exit open toward the other side of the second chamber 130, in which the first chamber 110 is irradiated with the first plasma jet of each of the first plasma ejection tools 104 through the first chamber 110 and the second plasma jet of each of the second plasma ejection tools 185 through the second chamber 130, and diffuses a first sterilizing gas of the first plasma jet and a second sterilizing gas of the second plasma jet toward the first entrance and the first exit of the first chamber 110. The first sterilizing gas or the second sterilizing gas includes ozone and active oxygen.

Referring to FIGS. 2 and 3, the first chamber 110 includes a first entrance open toward one side of the second chamber 130, and a first exit open toward the other side of the second chamber 130, and further includes at least one ozone concentration measuring sensor 108 on the other sidewall of the first chamber 110 between the first entrance and the first exit of the first chamber 110, and a control unit (not illustrated) positioned on the outside of the second chamber 130 and electrically connected to the at least one ozone concentration measuring sensor 108 through the second chamber 130 to display a measured value of the at least one ozone concentration measuring sensor 108 on a monitor, in which the at least one ozone concentration measuring sensor 108 measures ozone concentrations from the first and second sterilizing gases diffused into the first chamber 110 through the first plasma jet of each of the first plasma ejection tools 104 and the second plasma jet of each of the second plasma ejection tools 185.

When the first chamber 110 is irradiated on the inside of the first chamber 110 with the first plasma jet from each of the first plasma ejection tools 104 and the first sterilizing gas of the first plasma jet fills the inside of the first chamber 110, referring to FIGS. 2 to 4, the second chamber 130 is irradiated at the gas inlet of the second chamber 130 with the second plasma jet of each of the second plasma ejection tools 185, and supplies the second sterilizing gas of the second plasma jet to the inside of the first chamber 110 through the gas inlet holes 124 of the second chamber 130.

Referring to FIGS. 2 and 3, the first chamber 110 or the second chamber 130 includes the first or second entrance open toward the one side of the second chamber 130, and the first or second exit open toward the other side of the second chamber 130, in which, when the first plasma ejection tools 104 or the second plasma ejection tools 185 supply the first sterilizing gas of the first plasma jet or the second sterilizing gas of the second plasma jet to the inside of the first chamber 110, referring to FIGS. 2 to 5, the second chamber 130 is filled with the first sterilizing gas and the second sterilizing gas diffused from the first entrance of the first chamber 110 toward the second entrance of the second chamber 130, and from the first exit of the first chamber 110 toward the second exit of the second chamber 130, and discharges the first and second sterilizing gases from the inside of the second chamber 130 toward the first entrance of the second chamber 130, from the inside of the second chamber 130 toward the second exit of the second chamber 130, and from the inside of the second chamber 130 toward each gas outlet holes 128 of the second chamber 130.

When each of the first plasma ejection tools 104 or each of the second plasma ejection tools 185 supplies the first sterilizing gas of the first plasma jet or the second sterilizing gas of the second plasma jet to the inside of the first chamber 110, referring to FIGS. 2 to 5, the second chamber 130 includes a filter (not illustrated) in each gas outlet hole 128 of the second chamber 130, and filters the first and second sterilizing gases through the filter, and discharges the filtered first sterilizing gas and the filtered second sterilizing gas toward the outside of the second chamber 130.

As illustrated in FIGS. 2 and 3, when viewed in the longitudinal direction of the second chamber 130, the first conveyer belt 150 is spaced apart from the second conveyer belt 170, and positioned at the same horizontal level as the second conveyer belt 170 or at a higher vertical level than the second conveyer belt 170. The first conveyer belt 150 includes a plurality of first support structures 140 below the first conveyer belt 150, as illustrated in FIGS. 2 and 3. Each of the first support structures 140 support both edges of the first conveyer belt 150 in the width direction of the first conveyer belt 150, as illustrated in FIG. 2.

As illustrated in FIGS. 2 to 4, the second conveyer belt 170 is rotated in the same rotational direction as the first conveyer belt 150, is formed to be smaller than the width of the first chamber 110, and covers each gas inlet hole 124 of the second chamber 130 while passing through the first chamber 110 and the second chamber 170. Referring to FIGS. 2 to 5, the second conveyer belt 170 is irradiated with the second plasma jet of each of the second plasma ejection tools 185 through each gas inlet hole 124 of the second chamber 130 and diffuses the second sterilizing gas of the second plasma jet toward the first chamber 110 through a mesh type belt 168.

Referring to FIGS. 1 and 2, the second chamber 130 includes a second entrance open toward one side of the second chamber 130, and a second exit open toward the other side of the second chamber 130, and as illustrated in FIG. 1, the second conveyer belt 170 includes an object detection sensor 164 positioned near the second entrance or second exit of the second chamber 130, and a control unit (not illustrated) positioned on the outside the second chamber 130 and electrically connected to the object detection sensor 164 to display the measured value of the object detection sensor 164 on the monitor, in which, referring to FIGS. 2 and 3, the object detection sensor 164 detects an object 190 positioned on the first conveyer belt 150 or the second conveyer belt 170, and the rotation of the second conveyer belt 170 is performed when detecting the object 190, or the rotation of the second conveyer belt 170 is stopped when not detecting the object 190. In this case, the object 190 may be at least one of industrial and agricultural products, pharmaceuticals, and toys for children.

Referring to FIGS. 3 to 5, each of the second plasma ejection tools 185 includes a plasma nozzle 182 and a nozzle head 183 rotationally coupled in sequence to a plasma generator 181 positioned below the second chamber 130 and extending from the outside of the second chamber 130 toward the gas inlet hole 124 of the second chamber 130, and receives power from the outside of the second chamber 130 through a cable (not illustrated) to transform the air into the plasma in the plasma generator 181, receives compressed air from the outside of the second chamber 130 into the plasma generator 181, and passes the plasma and the compressed air through the plasma nozzle 182 and the nozzle head 183 to eject the plasma jet P from the nozzle head 183.

As illustrated in FIGS. 2 and 3, the second chamber 130 and the second conveyer belt 170 include a plurality of second support structures 90 below the second chamber 130 and the second conveyer belt 170, when viewed along the longitudinal direction of the second chamber 130. As illustrated in FIG. 2, each of the second support structures 90 supports both edges of the second chamber 130 or second conveyer belt 170 in the width direction of the second chamber 130 or the second conveyer belt 170.

When each of the first plasma ejection tools 104 and each of the second plasma ejection tools 185 supply the first sterilizing gas of the first plasma jet and the second sterilizing gas of the second plasma jet to the inside of the first chamber 110, as illustrated in FIGS. 2 and 3, the first conveyer belt 150 and the second conveyer belt 170 transfer the object 190 from the first conveyer belt 150 towards the second conveyer belt 170 during the driving of the first conveyer belt 150 and the second conveyer belt 170, and inside the first chamber 110, the object 190 is positioned below the plurality of first plasma ejection tools 104, and exposed to the first and second sterilizing gases on the second conveyer belt 170.

When each of the first plasma ejection tools 104 and each of the second plasma ejection tools 185 supply the first sterilizing gas of the first plasma jet and the second sterilizing gas of the second plasma jet to the inside of the first chamber 110, the first sterilizing gas and the second sterilizing gas increase the internal pressure of the first chamber 110 and the second chamber 130 compared to the atmospheric pressure on the outside of the second chamber 130 to block the inflow of external air from the outside of the second chamber 130 toward the inside of the second chamber 130.

As illustrated in FIG. 1, the second chamber 130 or the second conveyer belt 170 includes the plurality of second support structures 90 below the second chamber 130 or the second conveyer belt 170, when viewed along the longitudinal direction of the second chamber 130. As illustrated in FIG. 1, the plurality of second support structures 90 include a support plate 84 positioned between the plurality of second support structures 90 below the second chamber 130 and fixed to the plurality of second support structures 90 to collect foreign substances falling from the object 190 through the gas inlet holes 124 of the second chamber 130 while the object 190 is moved on the second conveyer belt 170.

### DESCRIPTION OF REFERENCE NUMERALS

| | | | |
|---|---|---|---|
| 90, 140: | support structure | 104: | first plasma ejection tool |
| 108: | ozone concentration measuring sensor | 110: | first chamber |
| 130: | second chamber | 150: | first conveyer belt |
| 164: | object detection sensor | 170: | second conveyer belt |
| 190: | object | | |

## Claims

1. A conveyer belt type plasma sterilizer comprising:
a first chamber having a shape of a square cylinder with a closed ceiling and an open bottom, the first chamber comprising a plurality of first plasma ejection tools on one sidewall;
a second chamber having a shape of a square cylinder, wherein the second chamber is formed to be longer than the first chamber to surround the first chamber when viewed along a longitudinal direction of the first chamber, and comprises a plurality of gas inlet holes below the first chamber and a plurality of gas outlet holes on the ceiling of the first chamber;
a first conveyer belt at one side of the second chamber, extending from an outside of the second chamber to a vicinity of the second chamber;
a second conveyer belt starting near the first conveyer belt, extending through the second chamber and the first chamber, and toward the other side of the second chamber; and
a plurality of second plasma ejection tools below the second chamber, facing each of the gas inlet holes of the second chamber, wherein
the first conveyer belt comprises a mesh type or non-mesh type belt, and
the second conveyer belt comprises a mesh type belt.

2. The conveyer belt type plasma sterilizer according to claim 1, wherein each of the first plasma ejection tools either includes a plasma nozzle and a nozzle head fixedly coupled in sequence to a plasma generator which penetrates the one sidewall of the first chamber and is exposed to an inside of the first chamber, or includes a plasma nozzle and a nozzle head rotationally coupled in sequence to the plasma generator.

3. The conveyer belt type plasma sterilizer according to claim 1, wherein each of the first plasma ejection tools includes a plasma nozzle and a nozzle head coupled in sequence to a plasma generator positioned on the one sidewall of the first chamber so as to extend from the second chamber toward the first chamber, and
receives power from the outside of the second chamber through a cable to transform an air into a first plasma in the plasma generator,
receives a compressed air from the outside of the second chamber to the plasma generator, and
passes the first plasma and the compressed air through the plasma nozzle and the nozzle head to eject a first plasma jet from the nozzle head.

4. The conveyer belt type plasma sterilizer according to claim 1, wherein each of the first plasma ejection tools includes a plasma nozzle and a nozzle head coupled in sequence to a plasma generator positioned on the one sidewall of the first chamber so as to extend from the second chamber toward the first chamber, and
irradiates an inside of the first chamber with a first plasma jet from the nozzle head to supply sterilizing gas of the first plasma jet to the first chamber to the inside of the first chamber during driving of the plurality of first plasma ejection tools, and
the sterilizing gas includes ozone and active oxygen.

5. The conveyer belt type plasma sterilizer according to claim 1, wherein the first chamber includes a first entrance open toward the one side of the second chamber, and a first exit open toward the other side of the second chamber,
the first chamber is irradiated with a first plasma jet of each of the first plasma ejection tools through the first chamber and a second plasma jet of each of the second plasma ejection tools through the second chamber, and diffuses a first sterilizing gas of the first plasma jet and a second sterilizing gas of the second plasma jet toward the first entrance and the first exit of the first chamber, and
the first sterilizing gas or the second sterilizing gas includes ozone and active oxygen.

6. The conveyer belt type plasma sterilizer according to claim 1, wherein the first chamber includes a first entrance open toward the one side of the second chamber, and a first exit open toward the other side of the second chamber, and further includes:
at least one ozone concentration measuring sensor on the other sidewall of the first chamber between the first entrance and the first exit of the first chamber; and
a control unit positioned on the outside of the second chamber and electrically connected to the at least one ozone concentration measuring sensor through the second chamber to display a measured value of the at least one ozone concentration measuring sensor on a monitor, wherein
the at least one ozone concentration measuring sensor measures ozone concentrations from first and second sterilizing gases diffused into the first chamber through a first plasma jet of each of the first plasma ejection tools and a second plasma jet of each of the second plasma ejection tools.

7. The conveyer belt type plasma sterilizer according to claim 1, wherein, when the first chamber is irradiated on the inside of the first chamber with a first plasma jet from each of the first plasma ejection tools and a first sterilizing gas of the first plasma jet fills the inside the first chamber,
the second chamber is irradiated at the gas inlet of the second chamber with a second plasma jet of each of the second plasma ejection tools and the second chamber supplies a second sterilizing gas of the second plasma jet to the inside of the first chamber through the gas inlet holes of the second chamber.

8. The conveyer belt type plasma sterilizer according to claim 1, wherein the first chamber or the second chamber includes a first entrance or a second entrance open toward the one side of the second chamber, and a first exit or a second exit open toward the other side of the second chamber, and
when the first plasma ejection tools or the second plasma ejection tools supply a first sterilizing gas of a first plasma jet or a second sterilizing gas of a second plasma jet to an inside of the first chamber,
the second chamber is filled with the first sterilizing gas and the second sterilizing gas diffused from the first entrance of the first chamber towards the second entrance of the second chamber, and from the first exit of the first chamber toward the second exit of the second chamber, and
discharges the first sterilizing gas and the second sterilizing gas from an inside of the second chamber toward the first entrance of the second chamber, from the inside of the second chamber toward the second exit of the second chamber, and from the inside of the second chamber toward each of the gas outlet holes of the second chamber.

9. The conveyer belt type plasma sterilizer according to claim 1, wherein, when each of the first plasma ejection tools or each of the second plasma ejection tools supplies a first sterilizing gas of a first plasma jet or a second sterilizing gas of a second plasma jet to an inside of the first chamber,
the second chamber includes a filter in each gas outlet hole of the second chamber, and filters the first sterilizing gas and the second sterilizing gas through the filter, and discharges the filtered first sterilizing gas and the filtered second sterilizing gas toward the outside of the second chamber.

10. The conveyer belt type plasma sterilizer according to claim 1, wherein, when viewed in a longitudinal direction of the second chamber, the first conveyer belt is spaced apart from the second conveyer belt and positioned at a same horizontal level as the second conveyer belt or positioned at a higher vertical level than the second conveyer belt.

11. The conveyer belt type plasma sterilizer according to claim 1, wherein the first conveyer belt includes a plurality of first support structures below the first conveyer belt, and each of the first support structure supports both edges of the first conveyer belt in a width direction of the first conveyer belt.

12. The conveyer belt type plasma sterilizer according to claim 1, wherein the second conveyer belt is rotated in a same rotational direction as the first conveyer belt, is formed to be smaller than a width of the first chamber, and covers each gas inlet hole of the second chamber while passing through the first chamber and the second chamber.

13. The conveyer belt type plasma sterilizer according to claim 1, wherein the second conveyer belt is irradiated with a second plasma jet of each of the second plasma ejection tools through each gas inlet hole of the second chamber, and diffuses a second sterilizing gas of the second plasma jet toward the first chamber through the mesh type belt.

14. The conveyer belt type plasma sterilizer according to claim 1, wherein the second chamber includes a second entrance open toward the one side of the second chamber, and a second exit open toward the other side of the second chamber, and
the second conveyer belt includes an object detection sensor positioned near the second entrance or the second exit of the second chamber, and a control unit positioned on the outside of the second chamber and electrically connected to the object detection sensor to display a measured value of the object detection sensor on a monitor, wherein
the object detection sensor detects an object positioned on the first conveyer belt or the second conveyer belt, and
a rotation of the second conveyer belt is performed when detecting the object or the rotation of the second conveyer belt is stopped when not detecting the object.

15. The conveyer belt type plasma sterilizer according to claim 1, wherein each of the second plasma ejection tools includes a plasma nozzle and a nozzle head rotationally coupled in sequence to a plasma generator positioned below the second chamber so as to extend from the outside of the second chamber toward the gas inlet holes of the second chamber, and
receives power from the outside of the second chamber through a cable to transform an air into a plasma in the plasma generator,
receives a compressed air from the outside of the second chamber to the plasma generator, and
passes the plasma and the compressed air through the plasma nozzle and the nozzle head to eject a plasma jet from the nozzle head.
